# EUROPEAN PATENT SPECIFICATION

(11) **EP 1 596 791 B1**
(45) Date of publication and mention of the grant of the patent: **20.07.2011**
(21) Application number: 03816102.2
(22) Date of filing: 23.12.2003
(51) Int. Cl.: A61F 7/12

(54) **DELIVERING COOLED FLUID TO SITES INSIDE THE BODY**
ABGABE EINER GEKÜHLTEN FLÜSSIGKEIT AN STELLEN IM KÖRPERINNEREN
ADMINISTRATION D'UN FLUIDE REFROIDI A DES SITES A L'INTERIEUR DU CORPS

(30) Priority: 21.02.2003 US 372035
(43) Date of publication of application: 23.11.2005
(73) Proprietor: Boston Scientific Limited, St. Michael, Barbados, West Indies (BB)
(72) Inventor: DRASLER, William, J., Minnetonka, MN 55345 (US); HARRISON, Kent, Maple Grove, MN 55311 (US); JENSON, Mark, L., Greenfield, MN 55357 (US); KOKATE, Jaydeep, Y., Maple Grove, MN 55311 (US); RICHARDSON, Leonard, B., Brooklyn Park, MN 55444 (US)
(74) Representative: Peterreins, Frank
(86) International application number: PCT/US2003/041153
(87) International publication number: WO 2004/075796

(56) References cited:
- WO-A-00/69323
- WO-A-01/36035
- WO-A-97/06739
- US-B1- 6 517 533

## Description

### BACKGROUND

The flow of oxygenated blood through the coronary arteries may be reduced or completely blocked by a thrombus or embolus associated with an underlying narrowing of the artery, commonly referred to as a lesion, causing acute myocardial infarction (AMI). Evidence shows that early reperfusion dramatically reduces injury to an ischemic tissue region, that is, the tissue region deprived of oxygenated blood, as the injury to the tissue continues throughout the ischemic event. Thus, early treatment of the coronary blockage using, for example, percutaneous transluminal coronary angioplasty (PTCA) or lytic therapy is desirable. Once the lesion in the coronary artery is repaired, normal blood flow may be restored to the ischemic tissue region.

Reperfusion injury may occur upon the reestablishment of blood flow due to a number of factors including oxygen radical formation, microvascular plugging, inflammatory reactions, and metabolic disturbances. It is possible to reduce reperfusion injury to the ischemic tissue region by cooling the tissue before reperfusion. Mild cooling of the tissue region to a temperature of 33 degrees Celsius, which is approximately four degrees cooler than normal body temperature, provides a protective effect, likely by the reduction in the rate of chemical reactions and the reduction of tissue activity and associated metabolic demands. Although the target cooling temperature is 33 degrees, cooling the target tissue to between 28 and 36 degrees Celsius may provide benefit as well. There are also benefits to cooling the blood entering an ischemic zone, such as reducing platelet aggregation and neutrophil adhesion which decreases the likelihood of microvascular plugging.

One way an ischemic tissue region in the heart may be cooled is by placing an ice pack over the patient's heart. Another method involves puncturing the pericardium and providing cooled fluid to a reservoir inserted into the pericardial space near the ischemic tissue region. In another cooling method, the target tissue is directly perfused with a cooled solution. For example, a catheter having a heat transfer element located in the catheter's distal tip may be inserted into a blood vessel to cool blood flowing into and through the heart. It is also possible to cool the ischemic tissue region by supplying cool blood to the heart through a catheter placed in the patient's coronary sinus. WO 01/36035 discloses a catheter carrying a shunt with an occluder. US 6517533 shows a balloon catheter with working means. WO 97/06739 shows an ablation catheter.

### SUMMARY

The invention features a catheter system according to the claims. A catheter for infusing a fluid to a site internal to the body is provided. The catheter includes an elongated member having a distal end positionable to be near the internal site and a lumen extending longitudinally through the member to the distal end of the member. An element cools fluid as it flows through the lumen before the fluid exits the lumen at the distal end.

According to the invention, the lumen is formed to guide a second catheter, which is a dilation catheter. The elongated member of the catheter may have a distal portion that includes the element and is shaped for insertion into an aorta and into the ostium of a vessel. The catheter may also include a plurality of sub-elements that cool the fluid flowing through the lumen, and flexible tubing attached to the elongated member between the sub-elements. A temperature sensor to measure the temperature of fluid flowing through the lumen that has a sensing portion located near the distal end of the elongated member may also be provided.

The element may be a thermoelectric cooler having a plurality of thermoelectric semiconductors. The thermoelectric semiconductors may be electrically connected in a parallel configuration to permit the thermoelectric semiconductors to be powered by a single voltage source. The element may also be a sealed chamber that cools the fluid by using a Joule-Thompson orifice to create a phase change of a liquid to a gas inside the chamber. A temperature sensor monitors the temperature of the sealed chamber.

Implementations may also include a sealing balloon positioned near the distal end of the elongated member that seals an external surface of the elongated member with a wall of a vessel. At least one hole may be provided in the elongated member proximal of the element to permits blood to enter the lumen. The temperature sensors may also comprise thermocouples.

In embodiments, the lesion may be treated using an interventional catheter, which may be inserted through a lumen of a catheter that provides cooled fluid to the ischemic tissue region. Treatment of a lesion in a coronary artery and a coronary vein is provided. The fluid provided to the ischemic tissue region may be cooled as it flows through a lumen in a catheter. A temperature sensor may sense the temperature of the fluid provided to the ischemic tissue region.

The cooled fluid may be provided either before or after physiological blood flow is restored. Further, the providing of cooled fluid may occur for a period of time after the treatment of the lesion. Cooled fluid may also be provided to an ischemic tissue region located in the brain or in the kidney. Cooled fluid may be delivered to a tissue area adjacent to the ischemic tissue region before the treatment of the lesion, and may continue to be provided to the tissue area adjacent to the ischemic tissue region during and after the treatment of the lesion. In applications where the cooled fluid is blood, the blood may enter the lumen through at least one hole in the catheter that is located proximal to a region of the catheter that cools the blood.

In embodiments, the delivering of cooled blood may occur during the angioplasty procedure and may be delivered to the ischemic tissue region through the dilation catheter. The blood that is delivered to the ischemic tissue region may be cooled as it flows through the guide catheter. The guide catheter may also cool the fluid delivered through the dilation catheter. Further, the temperature of the cooled blood may be sensed by a temperature sensor.

The details of one or more embodiments of the invention are set forth in the accompanying drawings and the description below. Other features, objects, and advantages of the invention will be apparent from the description and drawings, and from the claims.

### DESCRIPTION OF DRAWINGS

FIG. 1 is a perspective view of a catheter that cools fluid for delivery to a site internal to the body.
FIG. 2A shows an alternative implementation of the catheter shown in FIG 1.
FIG. 2B shows an alternative implementation of the catheter shown in FIG. 1.
FIG. 3 is a cross-sectional view, in a longitudinal plane, of a portion of the catheter near the catheter's distal end.
FIG. 4 is a perspective view of a chilling section used for cooling fluid as it flows through the catheter.
FIG. 5 is a side view of the chilling section shown in FIG. 4.
FIG. 6 is a cross-sectional view, in a longitudinal plane, of a portion of the catheter containing a chilling section.
FIG. 7 is a cross-sectional view of the catheter along the line 7-7 shown in FIG. 6.
FIG. 8 is a cross-sectional view, in a longitudinal plane, of a portion of an alternative implementation of the catheter near the catheter's distal end.
FIG. 9 is a cross-sectional view of the catheter along the line 9-9 show in FIG. 8.
FIG. 10 is a cross-sectional view, in a longitudinal plane, of a portion of a dilation catheter near the catheter's distal end.
FIG. 11 shows the connection of the proximal ends of a guide catheter and a dilation catheter and the apparatus that may be required when the guide catheter and dilation catheter are used together to perform percutaneous transluminal coronary angioplasty (PTCA).
FIGS. 12-15 illustrate a method of performing a PTCA procedure to treat an ischemic tissue region caused by a lesion in a coronary artery.
FIG. 16 illustrates a method of treating an ischemic tissue region caused by a lesion in a coronary artery.

Like reference symbols in the various drawings indicate like elements.

### DETAILED DESCRIPTION

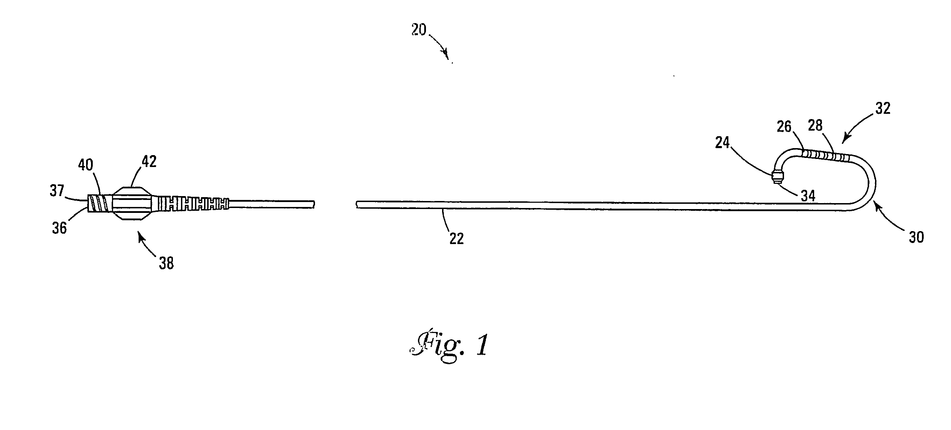

Referring to FIG. 1, a catheter 20 includes an elongate tubular shaft 22 with several chilling sections 26 in the shaft 22 near a distal end 34. The catheter 20 may be used in conjunction with an interventional catheter (not shown) to repair a lesion in a coronary artery that has reduced or completely blocked the flow of oxygenated blood to a tissue region. The lack of oxygenated blood causes the tissue region to become ischemic. The catheter 20 may be used to provide cooled fluid, such as blood, to the ischemic tissue region. The chilling sections 26 cool fluid flowing through the tubular shaft 22, and the cooled fluid exits the catheter's distal end 34. Delivery of cooled fluid to the ischemic tissue region reduces injury associated with the reperfusion of blood to the region.

The tubular shaft 22 is flexible to permit insertion into and through vessels in the body. In the implementation shown in FIG. 1, the shaft 22 has a U-shaped portion 30 near its distal end 34. This shape permits the distal end 34 of the catheter 20 to be inserted into the aorta, via a femoral artery, and seated in a coronary ostium to provide access to a coronary artery, as will be described later. Although the FIG. 1 implementation has a shaft 22 shaped for use in the heart, the shaft 22 may be constructed in other shapes appropriate for other applications, such as insertion into the carotid artery, the coronary sinus via the right atria, or the renal artery via the aorta.

The chilling sections 26 in this implementation are located near the catheter's distal end 34, and more specifically in a distal leg 32 of the shaft's U-shaped portion 30. The chilling sections 26 are cylindrically-shaped and are arranged in the shaft 22 such that the fluid flows longitudinally through the chilling sections 26 as the fluid flows through the shaft 22. In the FIG. 1 implementation, there are six chilling sections 26 that are spaced a small distance apart from one another. By way of example, each chilling section 26 is about one to ten millimeters long, and the spacing between the sections 26 is approximately the same distance. The length and spacing of the chilling sections 26 may depend upon, for example, the desired flexibility of the portion of the shaft 22 containing the chilling sections 26 and the amount of cooling necessary for the specific application. Flexible tubing 28 is attached to the shaft 22 between the chilling sections 26 to reinforce the portion of the shaft 22 containing the chilling sections 26 as it flexes to maneuver the distal end 34 through vessels in the body.

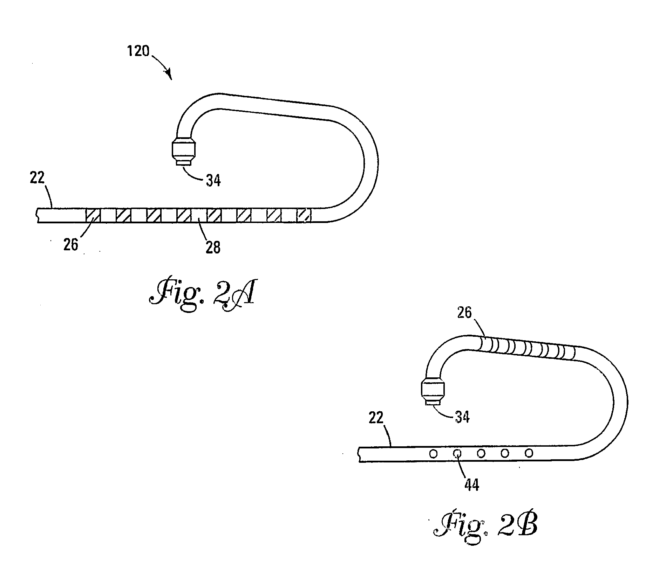

In other implementations, chilling sections 26 may be positioned elsewhere along the catheter's shaft 22. For example, in a different implementation shown in FIG. 2A, the chilling sections 26 in the catheter 120 are positioned farther from the catheter's distal end 34, but still nearer the distal end 34 than a proximal end of the shaft. Also, although there are six chilling sections 26 in the FIG. 1 implementation, there may be fewer or more chilling sections depending upon, for example, the volume of fluid being cooled, the location of the chilling sections 26 in the shaft 22, and the amount of cooling necessary for the specific application. For example, the FIG. 2A implementation has eight chilling sections 26.

Referring again to FIG. 1, a balloon 24 on the shaft 22 may be inflated to provide a seal between the catheter's distal end 34 and, for example, a coronary ostium. When the distal end 34 is seated in the coronary ostium, cooled fluid can be supplied to the ischemic tissue region via the coronary artery. The seal prevents cooled fluid delivered to the ischemic tissue region from escaping the coronary artery and entering the aorta, and at the same time, prevents warm blood in the aorta from entering the coronary artery, as will be discussed later. The balloon 24 in the implementation of FIG. 1 has a cylindrical-shaped outer surface when inflated, but could be constructed to take on different shapes as necessary depending on the shape of the location where a seal is to be made.

An adapter 38 is attached to the shaft 22 at a proximal end 36 of the catheter 20. The adapter 38 has a longitudinal opening 37 at the proximal end 36 to allow access to a lumen inside the shaft 22 (the lumen not being shown in FIG. 1). This internal lumen extends through the entire length of the shaft 22 to another longitudinal opening at the catheter's distal end 34. This lumen will be referred to as an infusion lumen, because the lumen is used to deliver, or infuse, cooled fluid to sites inside the body, as will be described in more detail later. The adapter 38 also includes an attachment portion 40 to attach devices such as a haemostatic adapter or a Y-adapter. The adapter 38 also includes a grip 42 where a physician holds and torques the catheter 20 if desired. In other implementations, different adapters 38 may be placed on the proximal end 36 of the catheter 20. For example, because the catheter 20 includes the sealing balloon 24, the adapter 38 may also include a second opening, or port, to provide access to an inflation lumen that extends longitudinally from the catheter's proximal end 36 to the balloon 24, as will be described in more detail later.

In the interventional procedure briefly described earlier, the catheter 20 is a guide catheter for a conventional dilation catheter used to perform a percutaneous transluminal coronary angioplasty (PTCA) (not shown in FIG. 1). Specifically, the dilation catheter is inserted through the guide catheter's proximal opening 37 in the proximal end 36 and into the internal infusion lumen described earlier. The dilation catheter is then extended through the shaft 22 so that the dilation catheter's balloon extends out of the distal end 34 of the shaft 22. As such, the dilation balloon may be placed at a lesion to be treated. After treatment of the lesion and removal of the dilation catheter from the guide catheter 20, fluid, such as blood, may be introduced into the infusion lumen through the proximal opening 37. This fluid flows through the infusion lumen and past the chilling sections 26 where the fluid is cooled, and ultimately is delivered to the ischemic tissue region.

In an alternative implementation shown in FIG. 2B, the catheter's shaft 22 may have one or a series of small holes 44 extending through the side of the shaft 22 and into the infusion lumen. The holes 44 may be located anywhere along the shaft 22 that is proximal of the chilling sections 26. When the catheter 20 is placed in a blood vessel, blood will be forced into the infusion lumen through the holes 44. Pressure exerted on the blood by the pumping of the heart forces the blood into the holes 44 and through the infusion lumen toward the distal end 34 of the catheter 20, where the blood is cooled by the chilling sections 26 and then delivered to the ischemic tissue region.

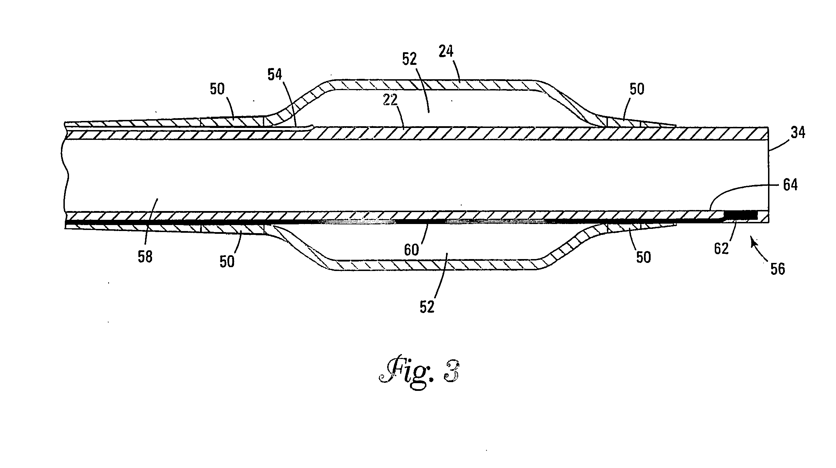

FIG. 3 shows a cross-sectional view, in a longitudinal plane, of a portion of the FIG. 1 catheter 20 near its distal end 34. As shown in FIG. 3, the sealing balloon 24 is positioned over the shaft 22, and around the shaft's entire circumference. Welds 50 secure and seal longitudinal ends of the balloon 24 to the shaft 22, thus forming a sealed chamber 52 between the shaft 22 and the balloon 24. An inflation lumen 54 extends through the shaft 22, from the adapter 38 at the catheter's proximal end 36 (shown in FIG. 1) to, and into, the balloon chamber 52 (FIG. 3). The balloon chamber 52 may be inflated and deflated by providing and removing an inflation medium (gas or liquid) into the chamber 52. As discussed previously, the balloon 24 provides a seal between the catheter shaft 22 and a vessel wall, for example, a coronary ostium. As such, the balloon 24 may be made of nylon, urethane, silicone, polyolefin copolymer, or other suitable materials. The materials of construction and dimensions of the balloon 24 may be different depending upon the application and the part of the body in which the balloon 24 is used.

FIG. 3 also shows a temperature sensor 56, located near the catheter's distal end 34, to measure the temperature of exiting cooled fluid. In this implementation, the temperature sensor 56 is a thermocouple. The thermocouple. 56 is made up of two conductive wires 60 of dissimilar material that are insulated from each other. The wires 60 extend longitudinally through the shaft 22, from the catheter's adapter 38 (shown in FIG. 1) to a location near the catheter's distal end 34. At this distal location, the conductive wires 60 are joined together to form a junction 62. The junction 62 has surface area that extends into an inner wall 64 of the shaft 22, such that the junction 62 is in thermal communication with fluid flowing through the infusion lumen 58 of the shaft 22. When two dissimilar conductors are joined in this manner, an electro-motive force (emf) is induced across the junction 62, the magnitude of which induced emf varies as a function of the junction's temperature. The induced emf may be measured at the proximal ends of the conductive wires 69 (that is, outside the patient), and thus it is possible to determine the temperature of the fluid flowing through the infusion lumen 58 just before it exits the catheter's distal end 34. If the fluid is not a desired temperature, then the chilling sections 26 may be adjusted to achieve the desired temperature, as will be described later. In other implementations, the temperature sensor 56 may be a thermistor or other suitable temperature sensing mechanisms. Further, the temperature sensor 56 may be placed at a different location in the shaft 22 to measure the temperature of the fluid flowing through the infusion lumen 58.

The infusion lumen 58, part of which is shown in FIG. 3, extends from the catheter's proximal end 36 (FIG. 1) to its distal end 34. The diameter of the lumen 58 depends on the application. For example, if blood is infused through the lumen 58, the diameter of the lumen 58 needs to be large enough so that blood cells infused at the desired rate are not destroyed by the shear forces generated as they flow through the lumen 58. The lumen diameter of various known guide catheters are sufficiently large to meet this requirement (e.g., 0.076" to 0.110"). In addition, if it is intended that blood be infused through the lumen 58 during the same time that a dilation catheter is in the lumen 58 (for example, if cooled blood is infused during a PCTA procedure), the diameter of the catheter's lumen 58 may need to be, in some cases, larger than the lumen diameter of a conventional guide catheter. On the other hand, the maximum diameter of the lumen 58 is limited by the diameter of the body lumen into which the catheter 20 is to be inserted and the size of the incision through which the catheter 20 is inserted into the patient.

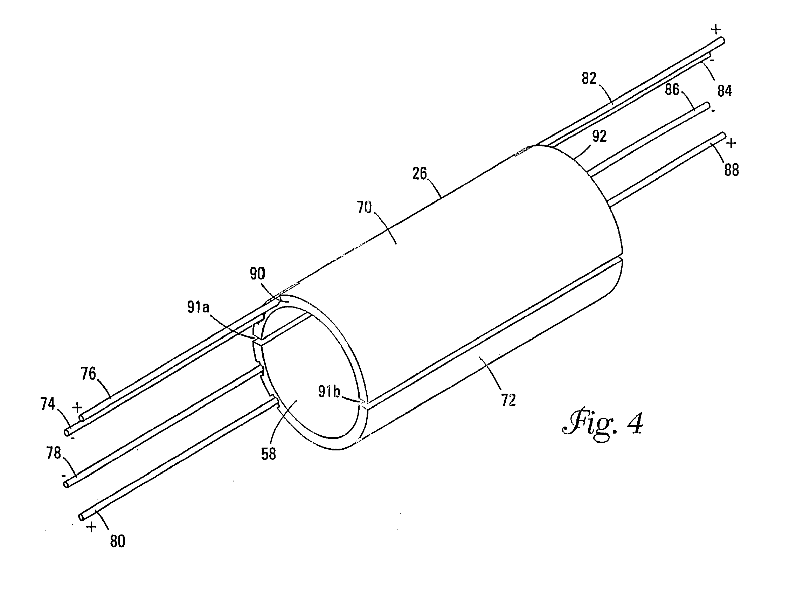

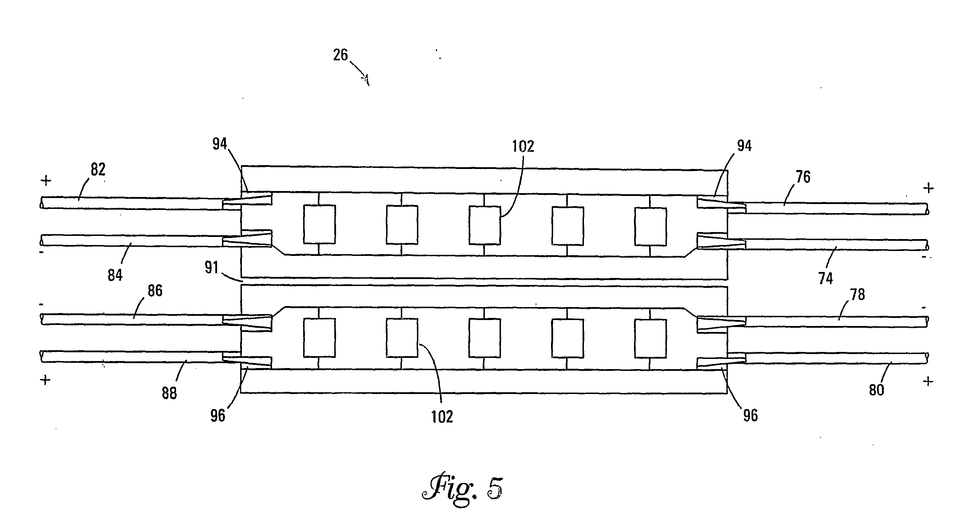

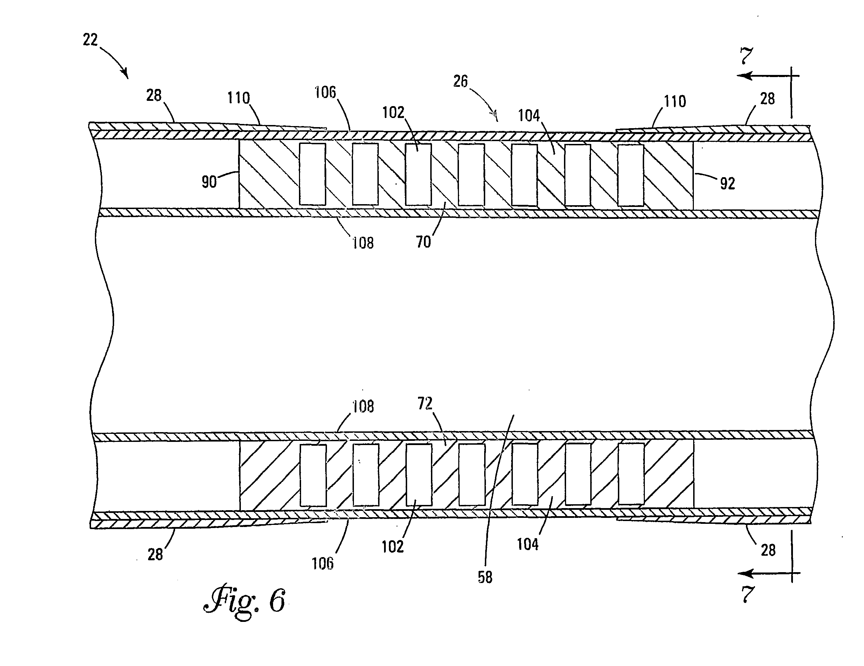

FIGS. 4-6 show an example of a chilling section 26 that may be used in the catheters shown in FIGS. 1 and 2. In this implementation, the chilling section 26 is a thermoelectric cooler (TEC). The TEC 26 cools the fluid flowing through the catheter 20 by using a thermal energy process known as the Peltier effect. To use this process, a low voltage DC power source may be applied to a thermoelectric module to move heat through the module from one side to the other, as will be described in detail later. FIG. 4 is a perspective view of the TEC 26. FIG. 5 is a side view of the TEC 26 that provides a simplified depiction of the thermoelectric semiconductor element pairs 102 that cool the fluid flowing through the catheter 20. FIG. 6 shows a cross-sectional view, in a longitudinal plane, of a portion of the catheter 20 containing the TEC 26 shown in FIGS. 4 and 5.

Referring to FIG. 4, the TEC 26 includes a first and second module 70 and 72, respectively. When the first and second modules 70 and 72 are placed together, they form a cylinder with lumen 58 through which fluid may flow. To form this cylinder-shaped structure, both the first and second modules 70 and 72 are in the shape of a half-cylinder, where the cylinder is split longitudinally into two equally-sized sections. The longitudinal edges of the first and second modules 70 and 72 are separated by small gaps 91a and 91b. The TEC 26 in this implementation may be, for example, one to ten millimeters long. Alternatively, the TEC 26 could be comprised of narrow flat modules or other shapes suitable for use in the catheter 20.

The first module 70 of the TEC 26 is connected to wires 74 and 76 at the first module's proximal end 90, and connected to wires 82 and 84 at the first module's distal end 92. In this implementation, wires 74 and 76 extend longitudinally through the shaft of the catheter toward the catheter's proximal end. The wires 74 and 76 may be connected to the first module 70 of another TEC 26 in the catheter located proximal to the TEC 26 shown in FIG. 6 (the connection not being shown in FIG. 6). If the TEC 26 is the most proximal chilling section in the shaft, the wires 74 and 76 extend longitudinally through the shaft to the catheter's proximal end for access outside of the patient. The wires 82 and 84 extend longitudinally through the shaft toward the catheter's distal end and may be connected to the first module 70 of another TEC 26 located distal to the chilling section shown in FIG. 6.

The second module 72 of the TEC 26 is similarly connected to wires 78 and 80 at the first module's proximal end 90, and connected to wires 86 and 88 at the first module's distal end 92. The wires 78, 80, 86, and 88 extend through the shaft and connect to the second modules 72 of the various TECs 26 in the catheter in the same manner as described for the first modules 70.

Referring to FIG. 5, the wires 74, 76, 82 and 84 are connected to the first module 70 at connection points 94. Similarly, the wires 78, 80, 86, and 88 are connected to the second module 72 at connections points 96. The first and second modules 70 and 72 include a number of thermoelectric semiconductor element pairs 102. The element pairs 102 in the first module 70 are powered by applying a DC voltage to the wires 74 and 76. Similarly, the element pairs 102 in the second module 72 are powered by applying a DC voltage to the wire 78 and 80. The element pairs 102 within the first and second modules 70 and 72 are arranged in a parallel configuration. Thus, the same DC voltage may be applied to all of the element pairs 102 in each of the modules 70 and 72. The wires 74 and 76 are connected to the wires 82 and 84 through the first module 70. This connection allows the DC voltage applied to the first module 70 to be applied to all of the first modules 70 in the catheter 20. As a result, all of the element pairs 102 in the first modules may be controlled with a single voltage source. Similarly, the wires 78 and 80 are connected to wires 86 and 88, which allows all of the element pairs 102 in the second modules 72 to be powered by a single voltage source. In other implementations, the modules 70 and 72 may be arranged in a series configuration. Further, the element pairs 102 may also be arranged in a series configuration within the modules 70 and 72.

Referring to FIG. 6, the element pairs 102 in the TEC are spaced throughout the first and second modules 70 and 72 of the TEC 26 and are packaged within an electrical insulator 104. In this implementation, the element pairs 102 include an n-type semiconductor and a p-type semiconductor electrically connected in series (the semiconductors not being shown). However, the semiconductors may be replaced with other suitable materials. The conductors are arranged in a substrate that electrically insulates the semiconductors within the element pairs 102 from heat sinks attached to the substrate on two sides of the element pairs 102 (the substrate and heat sinks not being shown). The element pairs 102 are arranged so that one heat sink is adjacent to an internal surface 108 of the first and second modules 70 and 72, and the other heat sink is adjacent to an external surface 106.

Applying the DC voltage to the modules 70 and 72 causes a current to pass through the n-type and p-type semiconductors within the element pairs 102. The current causes heat to be drawn from the heat sink near the internal surface 108 to the heat sink near the external surface 106. Through this process, the internal surface 108 is cooled, and at the same time, the external surface 106 is heated. By cooling the internal surface 108 of the first and second modules 70 and 72, fluid passing through the lumen 58 may also be cooled.

The cooling of the internal surfaces 108 may be adjusted by changing the voltage applied to the modules 70 and 72, which changes the current flowing element pairs 102. For example, if the current is increased, the cooling of the TEC 26 may be increased, which in turn further decreases the temperature of the fluid flowing through the lumen 58. Similarly, decreasing the current flowing through the element pairs 102 decreases the cooling of the TEC 26.

A flexible tubing 28 may be attached to the area of the shaft 22 proximal to the TEC 26 at a longitudinal end by welds 110. Alternatively, the flexible tubing 28 may be attached to the shaft 22 like a sleeve over the entire area of the shaft 22 containing the TECs 26. The flexible tubing 28 may be constructed of a polymer or a metal braid with polymer encapsulation depending upon the longitudinal length of the TEC 26. As described earlier, the flexible tubing 28 reinforces the area of the shaft 22 between the rigid TEC 26 as that area is flexed to maneuver the distal end of the catheter through vessels in the body. In implementations where the chilling sections 26 are flexible, the flexible tubing 28 may be omitted.

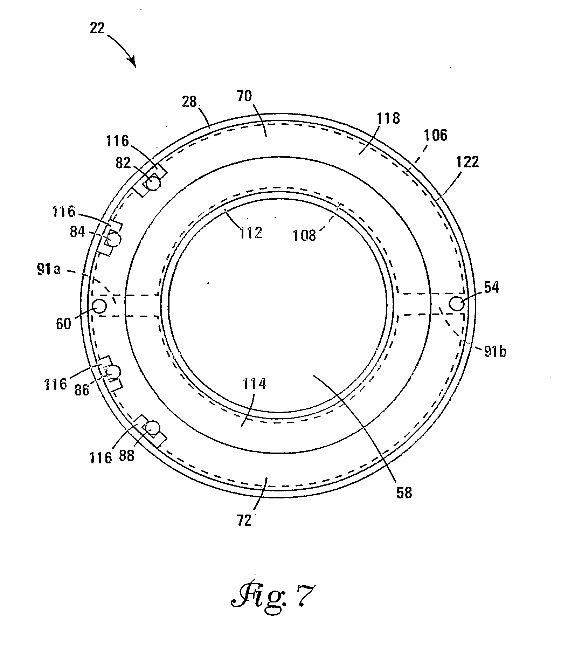

FIG. 7 shows a cross-sectional view of the catheter shaft 22 at line 7-7 of FIG. 6 looking toward the chilling section 26. In the implementation shown, the shaft 22 includes three primary layers 112, 114, and 118. An inner layer 112 encloses the infusion lumen 58 within, and is comprised of PTFE or FEP, as is conventional. A middle layer 114 encloses the inner layer 112 and is comprised of braided metal wires constructed of stainless steel or tungsten. An outer layer 118 enclosing the middle layer 116 is constructed of a polymer, such as nylon. In other implementations, different materials may be used to construct the layers 112, 114, and 118 of the catheter shaft 22, such as urethane or tantalum wire.

Also shown in FIG. 7 is the layer 28 of flexible tubing shown in FIG. 6. This flexible tubing layer 28 surrounds the shaft's outer layer 118 between the chilling sections 26. Dashed lines have also been added to the cross-section of FIG. 7 to indicate the location of the chilling sections 26 in the shaft 22 of the catheter with respect to the layers 112, 114, and 118. In this implementation, the first and second modules 70 and 72 are positioned between the shaft's inner layer 112 and its outer layer 118 such that the internal surfaces 108 of the first and second modules 70 and 72 are in thermal contact with the fluid flowing through the infusion lumen 58.

The wires 82, 84, 86, and 88 extend through the catheter shaft 22 in the layer 118 and are held in place by wire holders 116. In addition, the thermocouple wires 60 and the inflation lumen 54 extend from the distal end to proximal end of the catheter shaft 22 through layer 118 near the outer edge 122. The thermocouple wires 60 pass through the gap 91a between the first and second modules 70 and 72. Similarly, the inflation lumen 54 passes through the gap 91b.

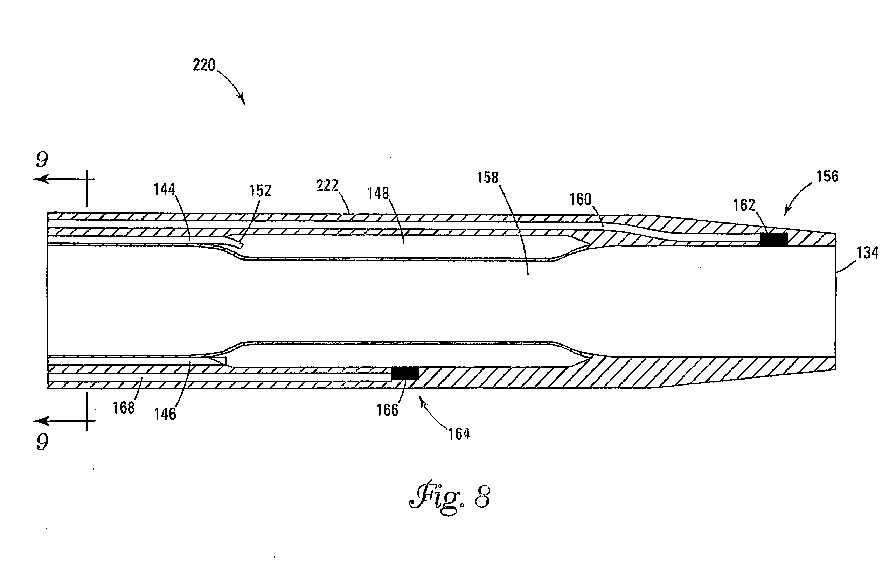

FIG. 8 shows a cross-sectional view, in a longitudinal plane, of a distal part of another catheter 220 that uses the physical process known as the Joule-Thompson effect to cool the fluid as it flows through the catheter 200. To use this process, a fluid is introduced into the thermo cooler chamber 148 and is allowed to change phase to a gas, which reduces the temperature of the thermo cooler chamber 148 and the fluid flowing through the catheter in thermal contact with the chamber 148. Like the catheter 20 described previously, the catheter 220 may be used in conjunction with an interventional catheter, such as a dilation catheter (not shown), to provide cooled fluid to an ischemic tissue region.

The catheter 220 includes a thermo cooler chamber 148 extending around the circumference of the catheter 220, an infusion tube 144, and an exhaust tube 146. The exhaust tube 146 removes the contents of the area 148 to maintain an ambient pressure in chamber 148. A highly-pressurized fluid, such as CO₂, N₂O, N₂, or He, enters the chamber 148 via the infusion tube 144 and an orifice 152. As the fluid changes phase from liquid to gas in the thermo cooler chamber 148, energy in the form of heat is pulled from the surrounding area, which cools the thermo cooler chamber 148 and the fluid flowing through the infusion lumen 158 of the catheter 220.

The thermo cooler chamber 148 may be, for example, one to 30 centimeters in length longitudinally and approximately 0.5 to three millimeters in width. These dimensions may be increased or decreased depending on factors, such as the amount of cooling desired and the pressure of the gas to be introduced to the thermo cooler chamber 148. The walls of the thermo cooler chamber 148 are noncompliant but flexible to accommodate the pressure changes caused by the introduction and removal of gas into the chamber 148. In this implementation, the walls are made of PET, but could be constructed of any material with similar properties, such as nylon. Further, the thermo cooler chamber 148 could be placed at different locations in the shaft 222 to cool the fluid flowing through the infusion lumen 158. The cooler chamber 148 may be coated with a polymer to insulate its exterior from the heat of the body (not shown). Alternatively, a layer of CO₂ may be introduced into a separate exterior pocket surrounding the cooler chamber 148 to provide insulation (not shown).

The exhaust tube 146 extends through the catheter shaft 222 from the thermo cooler chamber 148 to the proximal end of the catheter 220 (not shown). The infusion tube 144 also extends through the catheter shaft 222 from the thermo cooler chamber 148 to the proximal end of the catheter 220. The distal end of the infusion tube 144 may include one or more orifices 152 to control the flow of fluid into the thermo cooler chamber 148. In other implementations, the infusion tube 144 may be shaped differently to direct the flow of the fluid to the chamber 148.

A temperature sensor 164 is located near the thermo cooler chamber 148 and monitors the temperature of the chamber 148. FIG. 8 also shows a temperature sensor 156 located near the catheter's distal end 134 to measure the temperature of cooled fluid as it exits the infusion lumen 158. In this implementation, the temperature sensors 156 and 164 are thermocouples. As described previously, the thermocouples 156 and 164 are made up of two conductive wires of dissimilar material and insulated from each other. The conductive wires are joined together to form junctions 162 and 166. The junction 162 is in thermal contact with the fluid flowing through the infusion lumen 158 of the shaft 222, and the junction 166 is in thermal contact with the expanding gas in the thermo cooler chamber 148. In other implementations, temperature sensors other than a thermocouple may be used, such as thermistors or other suitable temperature sensing mechanisms.

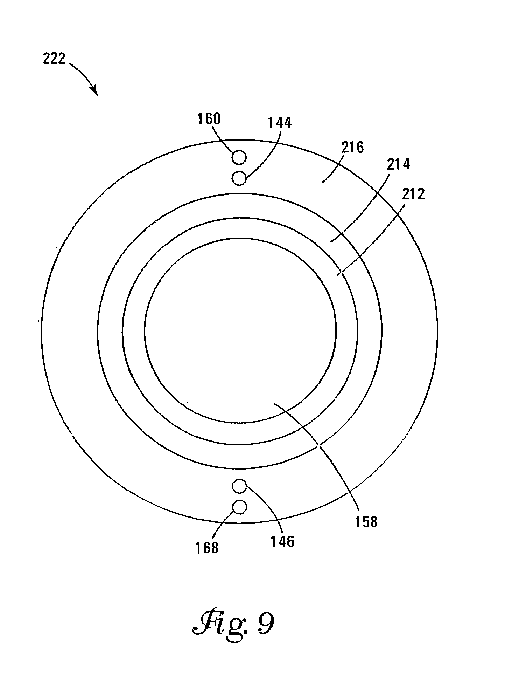

FIG. 9 shows a cross-sectional view of the catheter shaft 222 at line 9-9 of FIG. 8 looking away from the thermo cooler chamber. In the implementation shown, the shaft 222 includes three primary layers 212, 214, and 216. The inner layer 212 encloses the infusion lumen 158 within, and is comprised of PTFE or FEP as is conventional. A middle layer 214 encloses the inner layer and is comprised of braided metal wires constructed of stainless steel or tungsten. An outer layer 216 encloses the middle layer 214 and is constructed of polymer. In other implementations, different materials may be used to construct the layers 212, 214, and 216 of the catheter, such as urethane or tantalum wire.

The wires 160 for the thermocouple 156, the wires 168 for thermocouple 164, the infusion tube 144, and the exhaust tube 146 extend longitudinally through the catheter shaft 222 to the proximal end of the catheter (not shown) in the layer 216. In this implementation, the wires 160 attached to the thermocouple 156 are positioned in layer 216 near the infusion tube 144. Similarly, the thermocouple wires 168 attached to the temperature sensor 164 are located near the exhaust tube 146 in a position 180 degrees from the thermocouple wires 160 and infusion tube 144. In other implementations, the thermocouple wires 160 and 168, the infusion tube 144, and the exhaust tube 146 may be positioned in a different layer of the catheter shaft 222, or in a different position within the layer 216 shown in FIG. 9.

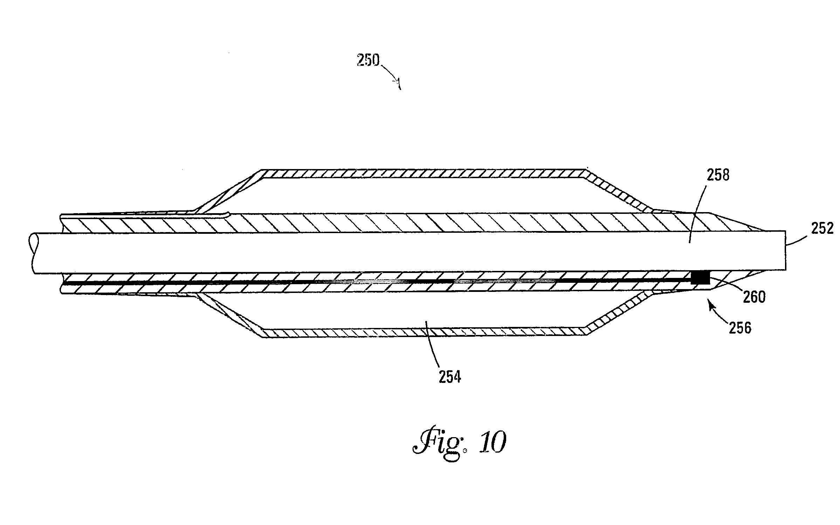

FIG. 10 shows a cross-sectional view, in a longitudinal plane, of a portion of a dilation catheter 250 near the catheter's distal end 252 that contains a temperature sensor 256. The catheter 250 may used in conjunction with a guide catheter, such as catheters 20, 120, or 220 to perform an interventional procedure, such as a PTCA procedure, to repair a lesion in a coronary artery that has reduced or completely blocked the flow of oxygenated blood to a tissue region. The catheter 250 may be inserted into and through the guide catheter to access the lesion in the coronary artery. The distal end 252 may then be placed through the lesion to provide cooled fluid, such as a saline, to the ischemic tissue region. The delivery of cooled fluid may continue until the dilation balloon 254 is inflated, the lesion has been repaired, and the catheter 250 has been removed from the coronary artery.

The temperature sensor 256 located near the catheter's distal end 152 measures the temperature of the fluid exiting the catheter for delivery to the tissue region. In this implementation, the temperature sensor 256 is a thermocouple. As described previously, the thermocouple 256 includes a junction 260 that has a surface area in thermal contact with fluid flowing through the infusion lumen 258 of the catheter 250. If the fluid is not a desired temperature (for example, 20 degrees Celsius in the case of cooling of ischemic tissue), then the temperature may be adjusted as desired. In other implementations, temperature sensors other than a thermocouple may be used, such as thermistor or other suitable temperature sensing mechanisms. Further, the temperature sensor 256 may be placed at a different location in the catheter 250 to measure the temperature of the fluid flowing through the infusion lumen 258.

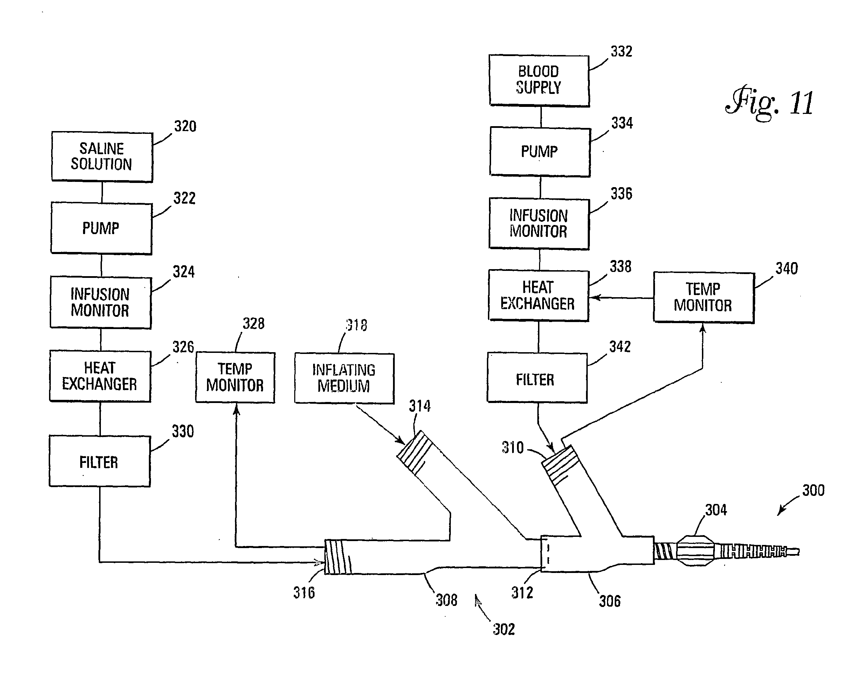

FIG. 11 shows various external devices that may be utilized when a conventional guide catheter 300 and an interventional catheter, such as a dilation catheter 302, are used together to deliver cool fluid to a site internal to the body. FIG. 11 also illustrates the configuration of the various adapters 304, 306, and 308 with respect to each other and the external devices in the system.

In a PTCA procedure, for example, a conventional Y-adapter 306 is attached to the adapter 304 at the proximal end of the conventional guide catheter 300. The Y-adapter 306 provides access to the infusion lumen of the guide catheter 300 through ports 310 and 312. The dilation catheter 302 is inserted into the infusion lumen of the guide catheter 300 through the port 312. The dilation catheter 302 may then be extended into and through the guide catheter 300 for access to the lesion that has reduced the blood flow in the coronary artery. In the configuration shown, a cooled fluid may be introduced to the infusion lumen of the guide catheter 300 through the port 310 for delivery to the ischemic tissue region.

The adapter 308 on the proximal end of the dilation catheter 302 includes two ports 314 and 316. The port 314 provides access to the dilation balloon on the dilation catheter 302. The dilation balloon may be inflated and deflated by providing and removing an inflation medium 314. Another port 316 provides access to the infusion lumen of the dilation catheter 302 so that cooled fluid may be delivered to a site internal to the body, for example, an ischemic tissue region.

In this implementation, the cooled fluid delivered by the dilation catheter 302 is a saline solution 320. The saline solution 320 may contain antioxidants or other vascular agents such as nitric oxide, lidocaine, nitroglycerine, insulin, adenosine, ATP, heat shock proteins, beta blockers, modifiers of calcium channel, modifiers of potassium channel, or other enzymes or metabolism modifiers. Modifiers of inflammatory response, modifiers of transmembrane transport, modifiers of lactic acid concentration, or other substances may also be included. The saline solution 320 could also contain delta opiod peptides (e.g. D-Ala2-Leu5-enkephalin DADLE) or other hibernation induction trigger agents. In other implementations, the saline solution 320 could be replaced with blood, a blood substitute, or a mixture of both. Further, the type of fluid provided to the ischemic tissue region through the dilation catheter 302 may be changed throughout the PTCA procedure.

The saline solution may be urged through the infusion lumen of the dilation catheter 302 by a conventional pump 322. For example, a positive displacement pump may be used to provide the pressure necessary to urge the saline solution 320 through the narrow infusion lumen of the dilation catheter 302. In other implementations the pump 322 may be replaced with a raised bag containing the saline solution 320 with an inflatable pressure cuff to control the infusion rate of the solution 320. A conventional infusion monitor 324 monitors the pressure and flow rate of the saline solution 320 through the infusion lumen of the dilation catheter 302. In the PTCA example, the saline solution 320 flows through the infusion lumen of the dilation catheter 302 at a rate of ten to 50 ml/min. The flow rate and pressure may be increased or decreased as required by different applications.

A heat exchanger may be used to cool the saline solution 320. A temperature monitor 328 may also be coupled to a temperature sensor, as described previously, to monitor the temperature of the solution 320 as it exits the distal end of the dilation catheter 302. Based on the feedback provided by the temperature monitor 328, the heat exchanger 326 may be adjusted to increase or decrease the temperature of the solution 320 to further reduce the tissue injury. The rate of tissue cooling may be controlled by adjusting either the infusion temperature, the infusion rate, or both. A filter 330 filters the solution 320 before it is introduced into the infusion lumen of the dilation catheter 302 for delivery.

The guide catheter 300 may also deliver a cooled fluid to a site internal to the body. In the PTCA example, the fluid delivered to the ischemic tissue is typically cooled blood 332. The blood 332 may be taken directly from the patient or may be from an external source. In the PTCA application and other applications in which the guide catheter 300 may be used, the blood 332 may be replaced with blood substitutes or saline solutions containing any of the agents and modifiers discussed previously.

In the PTCA example, a pump 334 urges the blood 332 through the infusion lumen of the guide catheter 300. For example, a roller pump may be used to provide blood to a coronary artery after a lesion has been repaired at a pressure normally applied by the heart. In other applications, other pumps may be used to increase or decrease the pressure of the fluid flowing through the infusion lumen as necessary. An infusion monitor 336 monitors the pressure and flow rate of the blood moving through the infusion lumen of the catheter 300.

A conventional heat exchanger 338 may be used to cool the blood 332 delivered to the ischemic region to a desired temperature, such as 33 degrees Celsius. A temperature monitor 340 may also be included to monitor the temperature of the blood 332 exiting the infusion lumen of the guide catheter 302. As described earlier, the heat exchanger 338 may be adjusted to increase or decrease the temperature of the solution 332 to minimize the tissue injury associated with an ischemic event. Further, the tissue cooling may be controlled by adjusting the flow rate of the solution 332 through the catheter 300. A filter 342 filters the blood 332 before it is introduced to the infusion lumen for delivery.

In an implementation in which the conventional guide catheter 300 is replaced with the guide catheter 20, 120, or 220 described previously, the blood 332 may be cooled inside the catheter, which eliminates the need for the heat exchanger 338. Further, in the implementation where the blood is introduced into the infusion lumen of the catheter 20 through small holes along the catheter shaft, the blood supply 332, the pump 334, the infusion monitor 336, and the filter 342 may not be needed. The only external apparatus that may be required in such an implementation is a temperature monitor attached to the temperature sensor to monitor the temperature of the blood exiting the infusion lumen and a device to control the cooling of the chilling sections in the catheter shaft. In an implementation in which the guide catheter includes a sealing balloon, another port on the proximal end of the catheter may be required to provide and remove an inflation medium to inflate and deflate the sealing balloon.

Further, in an implementation where guide catheter 300 is replaced with the guide catheter 20, 120, or 220, the fluid flowing though the dilation catheter 302 may be cooled by the guide catheters 20, 120, or 200. In an implementation such as this, the heat exchanger 326 may not be needed.

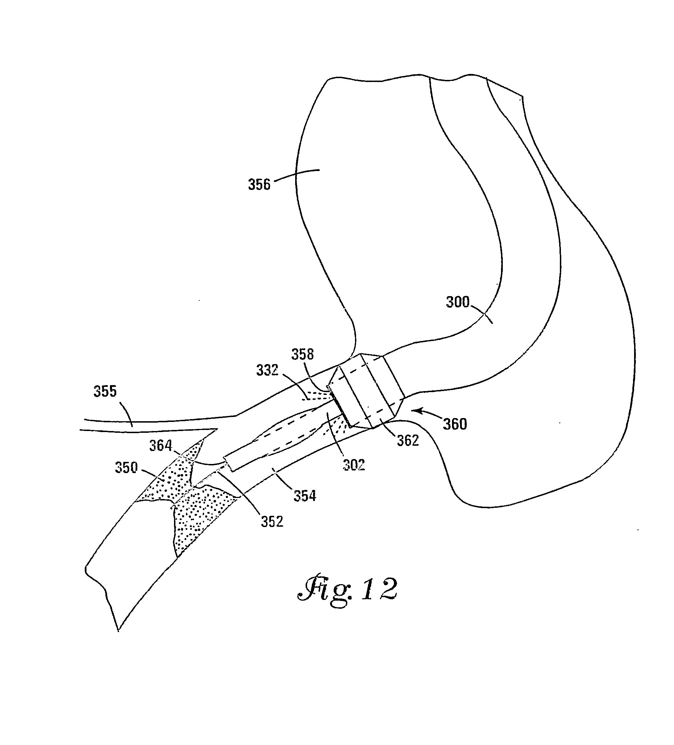

FIGS. 12-15 illustrate a method of performing a PTCA procedure to repair a lesion 350 in a coronary artery 354 that has reduced or completely blocked the flow of oxygenated blood to a tissue region 366 causing the tissue region to become ischemic. This method may be referred to as an "antegrade method" of performing a PTCA because the lesion 350 in the coronary artery 354 is accessed in the same direction as normal blood flow, i.e., from the aorta 356.

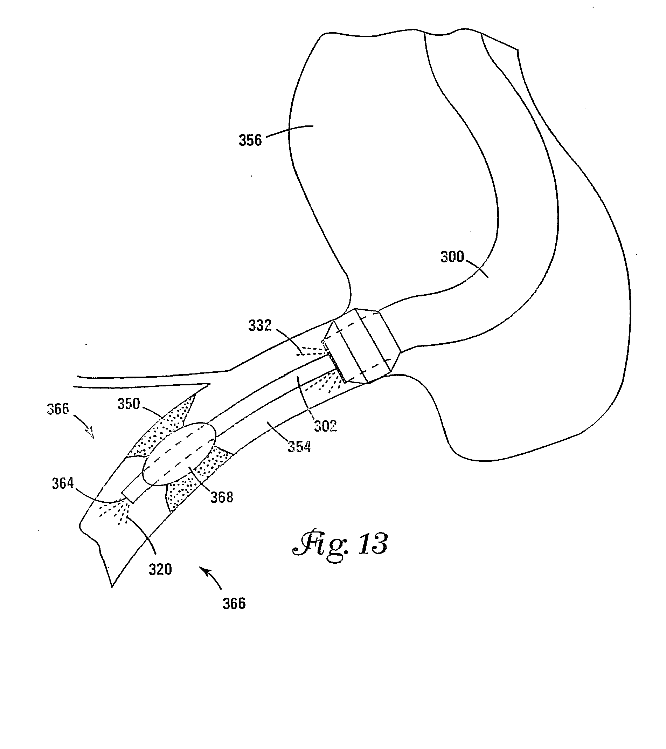

FIG. 12 shows a distal end 364 of the dilation catheter 302 extended through an opening in the distal end 358 of the guide catheter 300, which is seated in the coronary ostium 360. In the implementation shown, the guide catheter 300 includes a sealing balloon 362 that is inflated to provide a seal between the guide catheter's distal end 358 and the wall of the coronary artery 354. Once the distal end 358 of the guide catheter 300 is seated in the coronary ostium 360, cooled blood 332 may be delivered to the coronary artery 354, despite the fact that the coronary artery 354 is blocked by the lesion 350. The cooled blood provided by the guide catheter 300 may cool the tissue areas surrounding the ischemic tissue region 366 (shown in FIG. 13) via branching artery 355, which may provide a cooling effect on the ischemic tissue. To repair the lesion 350, the physician directs the distal end 364 of the dilation catheter 302 through the guide catheter 300 along the guide wire 352 into the coronary artery 354 and to a position distal to the lesion 350 as shown in FIG. 13.

Referring to FIG. 13, the dilation catheter's distal end 364 is positioned distal to the lesion 350 such that the catheter 302 may provide cooled fluid, such as the saline solution 320, to the ischemic tissue region 366. As described earlier, the saline solution 320 provided to the ischemic tissue region by the dilation catheter 302 may contain any number of chemical agents. Further, the contents of the saline solution 320 may be varied throughout the procedure. For example, a first solution may be used to provide an initial flush of the ischemic tissue region to rid the area of harmful free radicals or metabolic products that build up during the ischemic period. Once the initial flush is complete, a second solution may be provided to continue the cooling process. Additional solutions may be used throughout the procedure as desired.

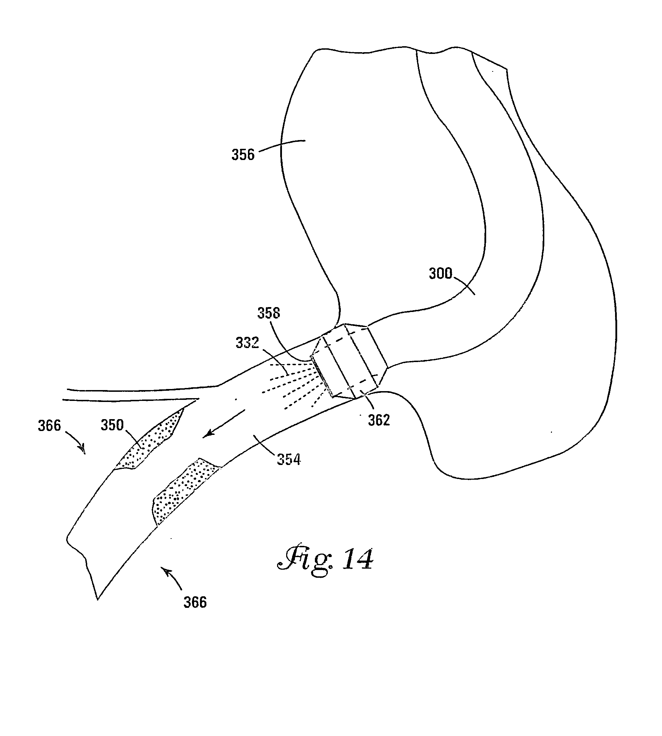

As the dilation catheter 302 is providing cooled fluid to the ischemic tissue region 366, the physician may inflate the dilation balloon 368 to repair the lesion 350. During the repair of the lesion 350, the dilation catheter may continue to deliver the cooled solution 320 to the ischemic tissue region 366. After the lesion 350 is repaired, the physician will then deflate the balloon 368 and remove the dilation catheter 302 from the coronary artery 354. The guide catheter 300 may continue to provide cooled blood 332 to the ischemic tissue region 366 for a period of time, for example twenty minutes, after the lesion 350 has been repaired, as shown in FIG. 14.

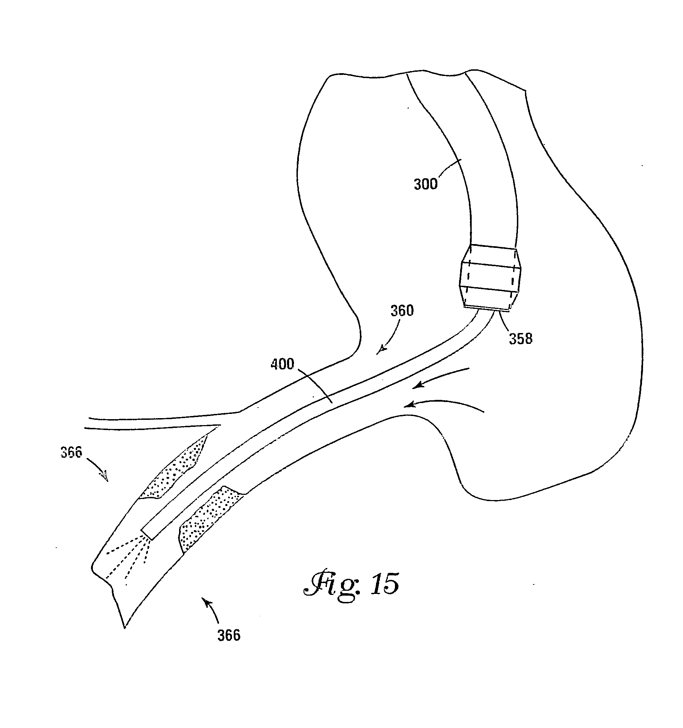

FIG. 15 shows the distal end of a subselective catheter 400 extending through an opening in the distal end 358 of the guide catheter 300. In this example, the distal end 358 of the catheter 300 is pulled back from the coronary ostium 360. The removal of the seal at the ostium 360 permits physiological blood flow to be restored, as indicated by the arrows. The catheter 400 may be used to infuse cooled blood or a cooled solution into a specific tissue region, such as the ischemic tissue region 366.

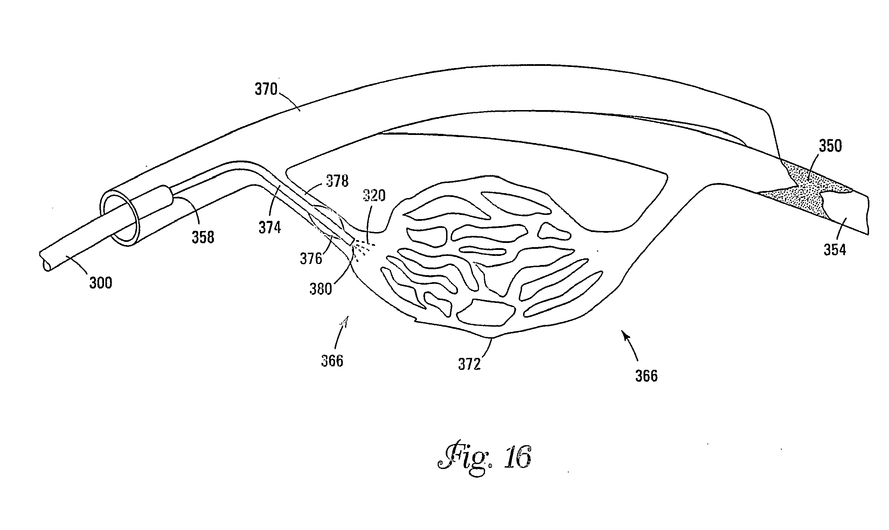

FIG. 16 shows a method of treating an ischemic tissue region caused by a lesion 350 that has reduced or completely blocked the flow of blood through the artery 354. The method in FIG. 16 may be referred to as a retrograde method of cooling an ischemic tissue region because the ischemic tissue region is accessed through a coronary vein 378 in a direction opposite normal blood flow.

A distal end 380 of a conventional sealing catheter 374 is extended through an opening in the distal end 358 of a conventional guide catheter 300, which is inserted into the coronary sinus 370. The distal end 380 of the sealing catheter 374 is positioned in the coronary vein 378 to provide a cooled solution to the capillary bed 372 for treatment of the ischemic tissue region 366. A sealing balloon 376 located near the distal end 380 may be inflated to prevent the cooled solution 320 provided by the sealing catheter 374 from flowing out of the coronary vein 378 and into the coronary sinus 370.

The cooled solution provided during the retrograde cooling method may contain arterial blood or an oxygen-carrying blood substitute. Alternatively, the cooled solution may contain any number of the chemical agents discussed previously. Further, the cooled solution may be changed throughout the procedure.

The retrograde cooling method shown in FIG. 16 may be used to cool an ischemic tissue region 366 in conjunction with the antegrade cooling method described previously to provide a more focused therapy. For example, the retrograde method could be used to target the ischemic tissue region 366, while the antegrade cooling method could be used to cool surrounding tissue. The methods could also be used in a sequential fashion. For example, the retrograde method could be used to initially cool the tissue prior to reperfusion and the antegrade method could be used at the time of reperfusion to give an added flush of the ischemic tissue region with the cooled solution to remove metabolic products that build up in the region during the ischemic event.

A number of implementations have been described. Nevertheless, it will be understood that various modifications may be made. For example, the devices and methods described may be used to cool other tissue, such as the brain, kidneys, and other organs in the body. Accordingly, other implementations are within the scope of the following claims.

## Claims

1. A catheter system comprising:
a dilation catheter (250; 302) having a balloon (254; 368);
a guide catheter (20; 120; 220; 300) for infusing a fluid to a site internal to the body, the guide catheter comprising:
an elongated member (22) having a proximal end (36), a distal end (34) positionable to be near the internal site, and a lumen (54) extending longitudinally from a proximal opening (37) in the proximal end (36) through the member (22) to an opening at the distal end (34) of the member;
a sealing balloon (24; 362) positioned near the distal end (34) of the elongated member (22) that is adapted to an external surface of the elongated member with
a wall of a vessel, and
an element (26) that cools fluid as it flows through the lumen (54) before the fluid exits the lumen at the distal end;
wherein the lumen (54) is formed to guide the dilation catheter and the dilation catheter (250; 302) is sized to be inserted through the proximal opening (37) and directed through the lumen (54) such that the dilation catheter balloon (254; 368) extends out of the opening distal end (34).

2. The catheter system of claim 1 wherein the elongated member comprises a distal portion that is shaped for insertion into an aorta and into an ostium of a vessel.

3. The catheter system of claim 1 wherein the element comprises a plurality of sub-elements to cool the fluid.

4. The catheter system of claim 3 further comprising flexible tubing attached to the elongated member between the sub-elements.

5. The catheter system of claim 1 wherein the element comprises a thermoelectric cooler.

6. The catheter system of claim 5 wherein the thermoelectric cooler comprises a plurality of thermoelectric semiconductors.

7. The catheter system of claim 6 wherein the thermoelectric semiconductors are electrically connected in a parallel configuration permitting the thermoelectric semiconductors to be powered by a single voltage source.

8. The catheter system of claim 1 wherein the element is located near the distal end of the elongated member.

9. The catheter system of claim 1 wherein the element comprises a sealed chamber that cools the fluid by using a Joule-Thompson orifice to create a phase change of a liquid to a gas inside the chamber.

10. The catheter system of claim 9 further comprising a temperature sensor that monitors the temperature of the sealed chamber.

11. The catheter system of claim 1 further comprising at least one hole in the elongated member proximal of the element the hole permitting blood to enter the lumen.

12. The catheter system of claim 1 further comprising a temperature sensor to measure the temperature of fluid flowing through the lumen.

13. The catheter system of claim 12 wherein the temperature sensor is a thermocouple.

14. The catheter system of claim 12 wherein the temperature sensor has a sensing portion located near the distal end of the elongated member.

## Patentansprüche

1. Kathetersystem, umfassend:
einen Dilatationskatheter (250; 302) mit einem Ballon (254; 368);
einen Führungskatheter (20; 120; 220; 300), um ein Fluid an eine Stelle innenliegend in dem Körpers zu infundieren, wobei der Führungskatheter umfasst:
ein längliches Element (22) mit einem proximalen Ende (36), einem distalen Ende (34), das in der Nähe der innenliegenden Stelle positionierbar ist, und einem Lumen (54), das sich longitudinal von einer proximalen Öffnung (37) an dem proximalen Ende (36) durch das Element (22) bis zu einer Öffnung an dem distalen Ende (34) des Elements erstreckt;
einen Dichtungsballon (24; 362), der in der Nähe des distalen Endes (34) des länglichen Elements (22) platziert ist, der angepasst ist, um eine externe Oberfläche des länglichen Elements mit einer Wand eines Gefäßes abzudichten, und
ein Element (26), das das Fluid kühlt, wenn es durch das Lumen (54) fließt, bevor das Fluid das Lumen am distalen Ende verlässt;
wobei das Lumen (54) gebildet ist, um den Dilatationskatheter zu führen und der Dilatationskatheter (250; 302) bemessen ist, um durch die proximale Öffnung (37) eingeführt zu werden und durch das Lumen (54) gelenkt zu werden, sodass der Dilatationskatheterballon (254; 368) sich aus der Öffnung am distalen Ende (34) erstreckt.

2. Kathetersystem gemäß Anspruch 1, wobei das längliche Element einen distalen Bereich umfasst, der geformt ist, um in eine Aorta und in ein Ostium eines Gefäßes eingeführt zu werden.

3. Kathetersystem gemäß Anspruch 1, wobei das Element eine Mehrzahl von Teilelementen umfasst, um das Fluid zu kühlen.

4. Kathetersystem gemäß Anspruch 3, weiter umfassend flexibles Rohrmaterial, das an dem länglichen Element zwischen den Teilelementen angebracht ist.

5. Kathetersystem gemäß Anspruch 1, wobei das Element einen thermoelektrischen Kühler umfasst.

6. Kathetersystem gemäß Anspruch 5, wobei der thermoelektrische Kühler eine Mehrzahl von thermoelektrischen Halbleitern umfasst.

7. Kathetersystem gemäß Anspruch 6, wobei die thermoelektrischen Halbleiter elektrisch parallel geschaltet sind, um den thermoelektrischen Halbleitern zu erlauben, durch eine einzige Spannungsquelle versorgt zu werden.

8. Kathetersystem gemäß Anspruch 1, wobei das Element in der Nähe des distalen Endes des länglichen Elements positioniert ist.

9. Kathetersystem gemäß Anspruch 1, wobei das Element eine abgedichtete Kammer umfasst, die das Fluid unter Benutzung einer Joule-Thompson-Düse kühlt, um eine Phasenänderung einer Flüssigkeit in ein Gas innerhalb der Kammer zu erzeugen.

10. Kathetersystem gemäß Anspruch 9, weiter umfassend einen Temperatursensor, der die Temperatur der abgedichteten Kammer überwacht.

11. Kathetersystem gemäß Anspruch 1, weiter umfassend zumindest ein Loch in dem länglichen Element proximal von dem Element, wobei das Loch erlaubt, dass Blut in das Lumen eintritt.

12. Kathetersystem gemäß Anspruch 1, weiter umfassend einen Temperatursensor, um die Temperatur von Fluid zu messen, das durch das Lumen fließt.

13. Kathetersystem gemäß Anspruch 12, wobei der Temperatursensor ein Thermokoppler ist.

14. Kathetersystem gemäß Anspruch 12, wobei der Temperatursensor einen Abtastbereich aufweist, der in der Nähe des distalen Endes des länglichen Elements platziert ist.

## Revendications

1. Système à cathéter, comprenant :
un cathéter de dilatation (250 ; 302) ayant un ballon (254 ; 368) ;
un cathéter de guidage (20 ; 120 ; 220 ; 300) pour la perfusion d'un fluide vers un site interne du corps, le cathéter de guidage comprenant :
un élément allongé (22) ayant une extrémité proximale (36), une extrémité distale (34) capable d'être positionnée pour être proche du site interne, et
un lumen (54) s'étendant longitudinalement depuis une ouverture proximale (37) dans l'extrémité proximale (36) à travers l'élément (22) jusqu'à une ouverture à l'extrémité distale (34) de l'élément ;
un ballon d'étanchement (24 ; 362) positionné proche de l'extrémité distale (34) de l'élément allongé (22), qui est adapté pour étancher une surface externe de l'élément allongé avec une paroi d'un vaisseau, et
un élément (26) qui refroidit du fluide lorsque celui-ci s'écoule à travers le lumen (54) avant que le fluide sorte du lumen à l'extrémité distale ;
dans lequel le lumen (54) est formé pour guider le cathéter de dilatation, et le cathéter de dilatation (250 ; 302) a une taille propre à être introduit à travers l'ouverture proximale (37) et dirigé à travers le lumen (54) de telle façon que le ballon (254 ; 368) du cathéter de dilatation s'étend hors de l'ouverture de l'extrémité distale (34).

2. Système à cathéter selon la revendication 1, dans lequel l'élément allongé comprend une portion distale qui est conformée en vue de l'insertion dans une aorte ou dans un ostium d'un vaisseau.

3. Système à cathéter selon la revendication 1, dans lequel élément comprend une pluralité de sous-éléments pour refroidir le fluide.

4. Système à cathéter selon la revendication 3, comprenant en outre des tubes flexibles attachés à l'élément allongé entre les sous-éléments.

5. Système à cathéter selon la revendication 1, dans lequel l'élément comprend un dispositif de refroidissement thermoélectrique.

6. Système à cathéter selon la revendication 5, dans lequel le dispositif de refroidissement thermoélectrique comprend une pluralité de semi-conducteurs thermoélectriques.

7. Système à cathéter selon la revendication 6, dans lequel les semi-conducteurs thermoélectriques sont connectés électriquement dans une configuration en parallèle permettant d'alimenter les semi-conducteurs thermoélectriques par une unique source de tension.

8. Système à cathéter selon la revendication 1, dans lequel élément est placé proche de l'extrémité distale de l'élément allongé.

9. Système à cathéter selon la revendication 1, dans lequel l'élément comprend une chambre scellée qui refroidit le fluide en utilisant un orifice de Joule-Thomson pour créer un changement de phase d'un liquide vers un gaz à l'intérieur de la chambre.

10. Système à cathéter selon la revendication 9, comprenant en outre un capteur de température qui surveille la température de la chambre scellée.

11. Système à cathéter selon la revendication 1, comprenant en outre au moins un trou dans l'élément allongé à proximité de l'élément, le trou permettant au sang d'entrer dans le lumen.

12. Système à cathéter selon la revendication 1, comprenant en outre un capteur de température pour mesurer la température du fluide s'écoulant à travers le lumen.

13. Système à cathéter selon la revendication 12, dans lequel le capteur de température est un thermocouple.

14. Système à cathéter selon la revendication 12, dans lequel le capteur de température comprend une portion sensible située proche de l'extrémité distale de l'élément allongé.
